# EUROPÄISCHE PATENTANMELDUNG

(11) **EP 1 226 781 A2**
(43) Veröffentlichungstag der Anmeldung: **31.07.2002**
(21) Anmeldenummer: 02000803.3
(22) Anmeldetag: 14.01.2002
(51) Int. Cl.: A61B 5/00

(54) **Madizinisches System zur Überwachung eines die Blutgerinnung betreffenden Messwertes eines Patienten**

(30) Priorität: 25.01.2001 DE 10103330
(71) Anmelder: SIEMENS AKTIENGESELLSCHAFT, 80333 München (DE)
(72) Erfinder: Christ, Tilo, 91058 Erlangen (DE); Hengerer, Arne, Dr., 91054 Erlangen (DE); Schmidt, Volker, Dr., 91054 Erlangen (DE); Striebel, Werner, 90592 Schwarzenbruck (DE)

(57) **Zusammenfassung**

Die Erfindung betrifft ein medizinisches System zur Überwachung eines die Blutgerinnung kennzeichnenden Messwertes eines Patienten und/oder zur Einstellung eines die Blutgerinnung beeinflussenden Medikamentes für einen Patienten in häuslicher Umgebung. Das System umfasst eine Vorrichtung (2) zur Erfassung von die Blutgerinnung betreffenden Messwerten des Patienten, eine Vorrichtung (2 bis 8) zur Übertragung der Messwerte an eine Systemzentrale (6), eine Vorrichtung (20 bis 22, 24) zur Abfrage der Messwerte und eine Empfangsvorrichtung (20 bis 22, 24) bei einer überwachenden Person, wobei die Systemzentrale (6) einen Speicher (15) für die Messwerte, eine Auswertevorrichtung (12) für die Messwerte zum Vergleich der Messwerte mit gespeicherten Sollwerten, einen Alarmgeber (12) zur Erzeugung eines Alarmsignals und eine Routingvorrichtung (14) zur Weiterleitung des Alarmsignals an eine Empfangsvorrichtung (3 bis 5, 20 bis 22, 24) einer zu alarmierenden Person einer Prozesskette aufweist.

## Beschreibung

Die Erfindung betrifft ein System zur Überwachung eines die Blutgerinnung kennzeichnenden Messwertes eines Patienten und/oder zur Einstellung eines die Blutgerinnung beeinflussenden Medikamentes für einen Patienten in häuslicher Umgebung.

Die Häufigkeit von kardiovaskulären Erkrankungen in der Bevölkerung nimmt stetig zu. Die Patienten müssen nach derartigen Erkrankungen, beispielsweise Herzinfarkten, häufig mit Bypässen, künstlichen Herzklappen oder künstlichen Blutgefäßen leben. Damit einhergehend besteht ständig die Gefahr von wiederkehrenden Thrombosen, Embolien oder Herzrhythmusstörungen, weshalb die Patienten meist lebenslang mit die Blutgerinnung beeinflussenden Medikamenten, sogenannten Antikoagulantien, wie Marcumar, Falithrom oder Warfarin, therapiert werden. Diese Therapie ist an sich hochwirksam, wird jedoch von einer Anzahl von Ärzten nicht durchgeführt, da der Medikamentenspiegel verhältnismäßig schwer einzustellen ist. Die Wirkung der Medikamente hängt nämlich entscheidend von der Ernährungsweise des jeweiligen Patienten sowie von der Einnahme weiterer Medikamente ab. Während bei einer Überdosierung die Gefahr von lebensbedrohlichen Blutungen, z.B. Nierenblutungen oder cerebralen Massenblutungen, besteht, können bei Unterdosierung Thrombosen, Infarkte oder Schlaganfälle nicht ausgeschlossen werden.

Die Überwachung eines die Blutgerinnung kennzeichnenden Messwertes eines Patienten sowie die Einstellung eines die Blutgerinnung beeinflussenden Medikamentes für einen Patienten in einer außerklinischen Umgebung ist derzeit nur unzureichend gelöst. Es existieren zwar Koagulationsmessgeräte, mit denen der Patient seine Blutgerinnungswerte zu Hause messen kann, welche er seinem behandelnden Arzt, beispielsweise papiergebunden oder als Fax meldet. Für eine adäquate Behandlung eines Patienten ist aber eine enge Kooperation mit einem Arzt erforderlich, insbesondere zur Kontrolle der Aktivität des Patienten, während der Neueinstellung oder Umstellung von Medikamenten, der Gabe zusätzlicher Medikamente und für akute Notfallsituationen.

Die Erfindung liegt daher die Aufgabe zugrunde, ein medizinisches System der eingangs genannten Art derart auszubilden, dass eine außerklinische Überwachung eines die Blutgerinnung kennzeichnenden Wertes eines Patienten und/oder eine außerklinische Einstellung eines die Blutgerinnung beeinflussenden Medikamentes für einen Patienten mit der für das gesundheitliche Wohl eines Patienten gebotenen medizinischen Sorgfalt erfolgen kann.

Nach der Erfindung wird diese Aufgabe gelöst durch ein medizinisches System zur Überwachung eines die Blutgerinnung kennzeichnenden Messwertes eines Patienten und/oder zur Einstellung eines die Blutgerinnung beeinflussenden Medikamentes für einen Patienten in häuslicher Umgebung mit einer Vorrichtung zur Erfassung von die Blutgerinnung betreffenden Messwerten des Patienten, einer Vorrichtung zur Übertragung der Messwerte an eine Systemzentrale, mit einer Vorrichtung zur Abfrage der Messwerte und einer Empfangsvorrichtung bei einer überwachenden Person, wobei die Systemzentrale einen Speicher für die Messwerte, eine Auswertevorrichtung für die Messwerte zum Vergleich der Messwerte mit gespeicherten Sollwerten, einen Alarmgeber zur Erzeugung eines Alarmsignals und eine Routingvorrichtung zur Weiterleitung des Alarmsignals an eine Empfangsvorrichtung einer zu alarmierenden Person einer Prozesskette aufweist. Mit diesem System kann die Überwachung von die Blutgerinnung eines Patienten betreffenden Messwerten aus dem Klinikbereich oder einer Arztpraxis herausgelöst und in die Lebensumgebung des Patienten verlagert werden. Insbesondere werden von dem System drei Größen überwacht: die Patientencompliance, die die Blutgerinnung betreffende Messwerte des Patienten und die Arztcompliance. Damit können für eine umfassende medizinische Betreuung des Patienten Alarme ausgelöst werden, wenn der Patient nicht oder zu häufig Messwerte erzeugt und übermittelt, wenn die Messwerte in einem gesundheitsgefährdenden Bereich liegen, so dass eine Notfallsituation bevorsteht oder bereits eingetreten ist, und wenn die alarmierte Stelle nicht adäquat reagiert.

Erforderliche Sofortmaßnahmen im Krankheitsfalle lassen sich unverzüglich einleiten, wenn der Alarmgeber derart ausgebildet ist, dass er ein Alarmsignal bei Über- oder Unterschreitung, insbesondere bei signifikanter Über- oder Unterschreitung, der Messwerte von gespeicherten Sollwertgrenzen erzeugt, das an eine Empfangsvorrichtung einer Person einer Behandlungskette bzw. Notfallkette geleitet wird.

Nach einer Variante der Erfindung kann der Alarmgeber derart ausgebildet sein, dass er ein Alarmsignal bei Ausbleiben von Messwerten erzeugt, das an eine Empfangsvorrichtung des Patienten geleitet wird, und/oder dass er ein Alarmsignal bei Ausbleiben einer Reaktionen einer Person der Behandlungskette auf besonders gekennzeichnete Messwerte erzeugt, das an eine Empfangsvorrichtung der Notfallkette geleitet wird.

Es hat sich als vorteilhaft erwiesen, wenn die Routingvorrichtung derart ausgebildet ist, dass das Alarmsignal an eine vorbestimmte, auswählbare Empfangsvorrichtung der Prozesskette geleitet wird oder dass das Alarmsignal durch automatisches Routing an eine von der Routingvorrichtung unter Berücksichtigung der Verfügbarkeit bestimmte Empfangsvorrichtung der Prozesskette geleitet wird.

Eine schnelle und effektive Benachrichtigung im Notfall lässt sich erreichen, wenn die Routingvorrichtung ein lernendes Expertensystem aufweist, das die Alarmierung der Prozesskette, beispielsweise die Verständigung des Hausarztes, Rufen des Notarztes, Organisieren des Transportdienstes und/oder Vorbereitung der Klinik, bewirkt.

Unerwünschte Fehlmeldungen werden reduziert bzw. ausgeschlossen, wenn die Auswertevorrichtung ein lernendes Expertensystem aufweist, das die Messwerte krankheits- und/oder problemspezifisch anhand von Regelsystemen oder auf Wahrscheinlichkeiten basierend interpretiert, aufgrund deren Ergebnis das Alarmsignal ausgelöst wird.

Eine Variante der Erfindung sieht vor, dass der Alarmgeber derart ausgebildet ist, dass er in Abhängigkeit von der Dringlichkeit einer Reaktion auf ein Ereignis unterschiedliche Alarmsignale erzeugt, die an Empfangsvorrichtungen einer Behandlungs- und/oder Notfallkette geleitet werden.

Ein Ausführungsbeispiel der Erfindung ist in den beigefügten schematischen Zeichnungen dargestellt. Es zeigen:
- Fig. 1: ein erfindungsgemäßes medizinisches System zur Überwachung eines die Blutgerinnung kennzeichnenden Messwertes eines Patienten und zur Einstellung eines die Blutgerinnung beeinflussenden Medikamentes für einen Patienten,
- Fig. 2: eine Benutzeroberfläche zur Konfiguration eines zentralen Alarmgebers, und
- Fig. 3: eine Benutzeroberfläche zur Konfiguration der Weiterleitung von Alarmen.

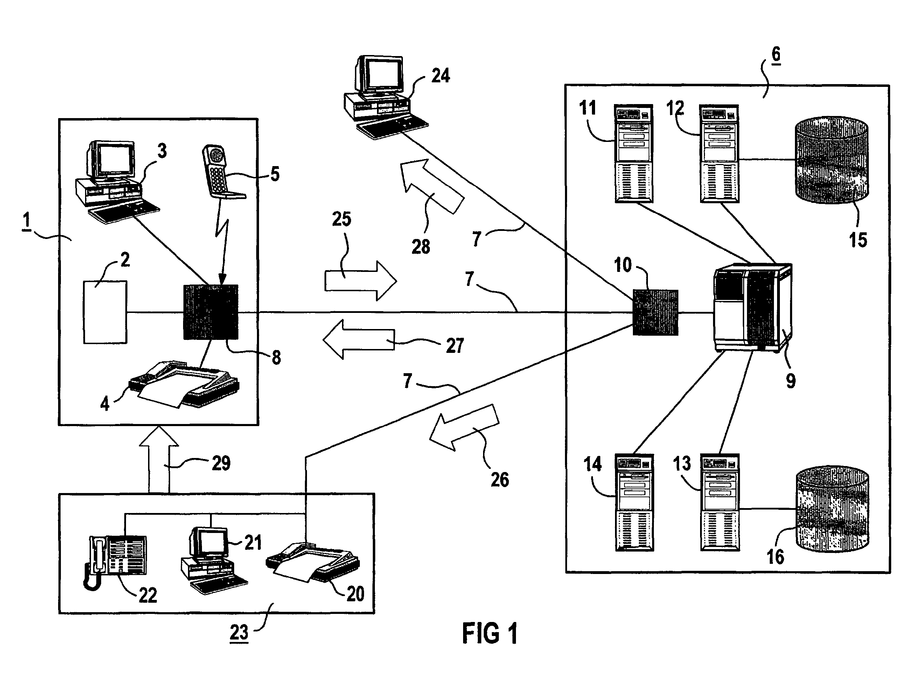

In der Fig. 1 ist ein erfindungsgemäßes System zur Überwachung von die Blutgerinnung betreffenden Messwerten eines nicht dargestellten Patienten und zur Einstellung eines die Blutgerinnung beeinflussenden Medikamentes für den Patienten in seinem Haus 1 dargestellt. Die Erfassung 2 der Messwerte, welche einerseits die Blutgerinnung des Patienten kennzeichnen und andererseits Auskünfte über die Art, Dosis und Einnahme von die Blutgerinnung beeinflussenden Medikamenten sowie über eingenommene Lebensmittel oder Farbe des Urins aufweisen, erfolgt teilweise automatisch und teilweise manuell durch den Patienten oder eine Pflegeperson. Hierzu wird ein sogenanntes POCT-Gerät 2 (point of care testing), welches auch als Koagulationsmonitor bezeichnet wird, verwendet. Dieses Gerät 2 ermöglicht es Blutgerinnungswerte, wie Quick oder PTT, des Patienten festzustellen sowie einen Selbsttest und eine Selbstdosierung eines Medikamentes, z.B. Warfarin, vorzunehmen. Derartige POCT-Geräte werden beispielsweise von der Firma November AG mit der Bezeichnung Novi Quick oder von der Firma Roche mit der Bezeichnung Coagucheck oder Coagucheck Plus vertrieben. Die die Eßgewohnheiten und die Farbe des Urins betreffenden Informationen können hingegen in einem Fragebogen erfasst werden.

Das vorzugsweise zu definierten Zeitpunkten zu erfassende Bündel 25 von Messwerten, welches anamnestische Daten zu eingenommenen Medikamenten, Messwerten zur Blutgerinnung und den Zeitpunkten der Messungen umfasst, kann nach entsprechender Eingabe bzw. Messung direkt von dem Gerät 2 oder über eine mit dem Gerät 2 verbundene Übertragungsvorrichtung für Informationen, beispielsweise per Personal Computer 3, per Faxgerät 4, per Telefon oder per Handy 5 an eine Systemzentrale 6 weitergeleitet werden. Dies kann über ein Kommunikationsnetz, beispielsweise über ein ISDN-Netz 7 erfolgen, an das die entsprechenden Endgeräte über ein ISDN-Interface 8 angeschlossen sind. Die Endgeräte können aber auch, soweit sie internetfähig sind, über ein entsprechendes Interface, wofür sich auch das ISDN-Interface 8 eignet, an das Internet zum Datentransfer angeschlossen sein.

Die Systemzentrale 6 dient zur Entgegennahme und Speicherung der Messwerte. Weiterhin soll sie eine Weiterverarbeitung der Messwerte und gegebenenfalls eine Auslösung von Alarmen bewirken.

In der Systemzentrale 6 ist hierzu ein Gateway 9 vorgesehen, das über ein ISDN-Interface 10 an dem ISDN-Netz 7 angeschlossen ist. An dem Gateway 9 kann ein Internet-Proxy-Server 11 zum Zugriff auf das Internet, eine Auswertevorrichtung 12 für die Messwerte, ein Patientendaten-Server 13 zur Verwaltung der Patientendaten und ein Kommunikations-Server 14 als Routingvorrichtung zur Zusammenarbeit aller Komponenten und Weiterleitung von Meldungen angeschlossen sein. Mit der Auswertevorrichtung 12 ist ein Datenspeicher 15 für die Messwerte und mit dem Patientendaten-Server 13 eine Datenbank 16 als Speichervorrichtung verbunden.

In der Systemzentrale 6 werden die Messwerte über das ISDN-Interface 10 eingelesen und in dem Datenspeicher 15 langfristig abgespeichert. Die Auswertevorrichtung 12 weist einen Komparator zum Vergleich der Messwerte mit in dem Datenspeicher 15 gespeicherten Sollwerten auf und wertet den Zeitpunkt der Übertragung der Messwerte aus. Außerdem umfasst die Auswertevorrichtung 12 einen Alarmgeber zur Erzeugung von Nachrichten und Alarmen bei abnormen Messwerten, beim Ausbleiben von Messwerten seitens des Patienten und beim Ausbleiben einer Reaktion seitens eines Arztes oder eines Patienten auf einen Alarm hin.

In der Systemzentrale 6 werden dabei alle Informationen zusammengeführt, die zur Aktivierung des Alarmgebers und zur Realisierung einer Weiterleitung von Alarmen und Nachrichten bei Abwesenheit des vorgesehenen Empfängers benötigt werden.

Mit der Systemzentrale 6 sind Empfangsgeräte wie beispielsweise ein Faxgerät 20, ein Personal Computer 21, ein Telefon 22 oder Handy verbunden, die beispielsweise zu einer Ärzte-Bereitschaft gehören und beispielsweise in einer Gemeinschaftspraxis 23 von Ärzten angeordnet sein kann.

Weiterhin ist im Falle des vorliegenden Ausführungsbeispiels ein Personal Computer 24 einer Praxis eines erstbehandelnden Arztes über das ISDN-Netz 7 mit dem Gateway 9 der Systemzentrale 6 verbunden. Eine Ausgabe der im Datenspeicher 15 gespeicherten Messwerte an eines der Endgeräte für Ärzte erfolgt über das ISDN-Interface 10.

Erfolgt die Erfassung des Bündels von Messwerten durch eine Pflegeperson, so ist für diese ein Personal Digital Assistant (PDA) oder ein Laptop vorgesehen, in dem alle Patientendaten eingegeben werden, die von diesem Pflegedienst besucht werden. Die Synchronisierung der Daten kann sofort, beispielsweise per Handy-Internet Verbindung, oder zu einem späteren Zeitpunkt erfolgen, wenn die Pflegeperson wieder im Büro oder in der Praxis eintrifft.

Wie bereits angedeutet wird ein Alarm bzw. ein weiterer Alarm durch den Alarmgeber der Auswertevorrichtung 12 vorzugsweise bei Vorliegen einer der Ereigniskonstellation wie das Ausbleiben von Messwerten seitens des Patienten, das Vorliegen abnormer Messwerte oder das Ausbleiben einer Reaktion auf einen generierten Alarme seitens der informierten Person, sei es ein Arzt oder der Patient selbst, ausgelöst. Die Auswertevorrichtung 12 beurteilt dabei, ob Messwerte oder Messwertkonstellationen als gesundheitsgefährdend einzustufen sind. Als Kriterium werden Grenzen aus der Literatur bzw. medikamentenspezifische Grenzwerte entnommenen, die in dem Datenspeicher 15 abgespeichert sind. Weiterhin können vom behandelnden Arzt für den Patienten individuelle Grenzen definiert werden.

Ein Alarm 27 an den Patienten wird beispielsweise dann ausgelöst, wenn fällig gewordene Messwerte vom Patienten nicht geliefert werden. Des weitern wird ein Alarm 26 aufgrund von einfachen und insbesondere von signifikanten Unter- bzw. Überschreitung der Messwerte von Sollwertgrenzen an die Endgeräte 20 bis 22 der Gemeinschaftspraxis 23 oder an den erstbehandelnden Arzt geleitet. In der Praxis kann dabei jeder behandelnde Arzt, welcher über eine entsprechende Berechtigung verfügt, die in dem Datenspeicher 15 gespeicherten Messwerte mit einem Personal Computer, einem Telefon, einem Fax einem Handy oder einer anderen Kommunikationseinrichtung abrufen.

Zur Prüfung der Messwerte und Auslösung von Alarmen kann ein Expertensystem eingesetzt werden, das die Messwerte krankheits- und problemspezifisch anhand von Regelsystemen oder auf Wahrscheinlichkeiten basierend interpretiert. Dabei ist eine Individualisierung des Expertensystems auf den Patienten vorgesehen, d.h. das Expertensystem lernt den Patienten, dessen Blutgerinnungswerte und die mit diesen in Zusammenhang stehenden Körperwerte es überwacht, im Überwachungsprozess immer besser kennen, es trifft dazu als lernendes System ständig Prognosen über zu erwartende zukünftige Messwerte, die es mit den wahren Messwerten vergleicht. Damit wird eine individualisierte Überwachung realisiert.

Im Fall komplexer Alarme kann ein Expertensystem eingesetzt werden, dass die Prozesse der Prozesskette, Erinnern des Patienten, Verständigung des Hausarztes, Rufen des Notarztes, Organisieren des Transportdienstes und/oder Vorbereitung der Klinik unter Berücksichtigung der Verfügbarkeit der Alarmempfänger übernimmt.

Die Prozesskette umfasst dabei die Gesamtheit der beteiligten Personen und Institutionen. Dazu gehören der Patient, die Behandlungskette mit Arzt, Pflege- und Transportdienst sowie die Notfallkette mit Feuerwehrleitstelle, Notfall- und Transportdienste sowie das Krankenhaus.

Alarme können in Abhängigkeit von der Dringlichkeit einer Reaktion einer benachrichtigten Person oder Institution in unterschiedlichen Dringlichkeitsstufen generiert werden, beispielsweise dringendst, dringend, Routine oder Standard. Welche Messwerte zu welchen Dringlichkeitsstufen führen, kann mit den gleichen Mechanismen wie die Beurteilung der Messwerte festgelegt werden und in dem Datenspeicher 15 abgespeichert sein.

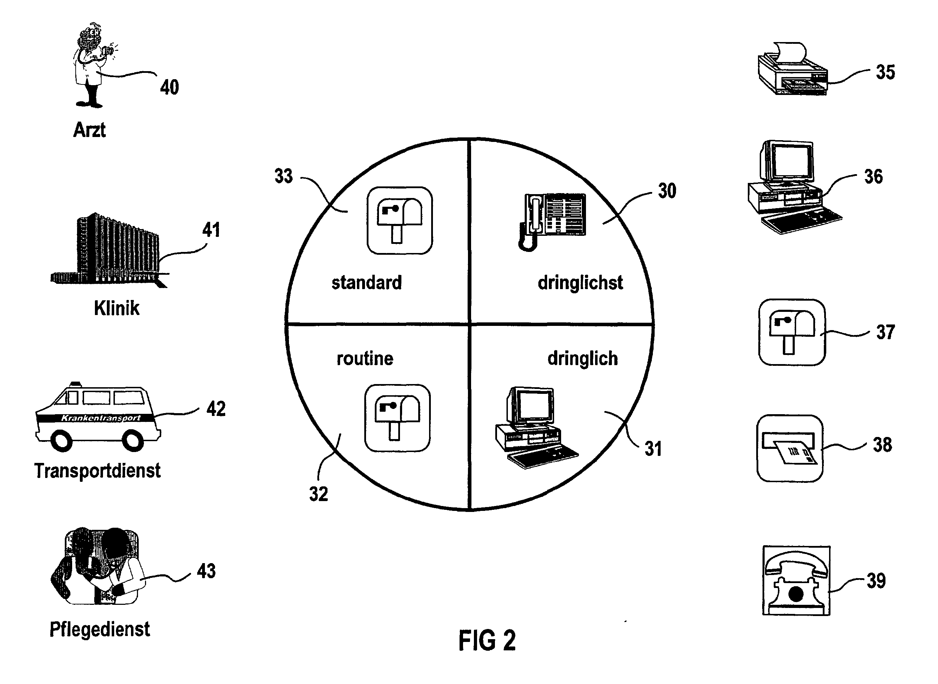

Die Konfiguration des zentralen Alarmgebers der Gemeinschaftspraxis 23 erfolgt durch den Anwender. Die Überwachungsinstanz wie einer der Ärzte legt für sich selbst fest, wie er Nachrichten unterschiedlicher Dringlichkeit erhalten will. Dazu verwendet er die in Fig. 2 gezeigte Benutzeroberfläche (user interface). Der Kommunikationskreis ist in Segmente 30 bis 33 unterteilt, die die Dringlichkeitsstufen der Alarmnachrichten repräsentieren.

Zur Konfiguration zieht der Arzt jeweils die Geräte aus der Reihe der Kommunikationsgeräte 35 bis 39 des Anwenders in die entsprechenden Segmente 30 bis 33, über die er bei Eintreffen einer klassifizierten Nachricht erreichbar sein möchte. Die Konfiguration wird in der Systemzentrale 6 gespeichert.

Bei der gewählten Konfiguration gemäß Fig. 2 wird der Arzt bei dringlichsten Fällen per Telefon 22, bei dringlichen Fällen per E-Mail über den Personal Computer 21 und in allen anderen Fällen per Post benachrichtigt.

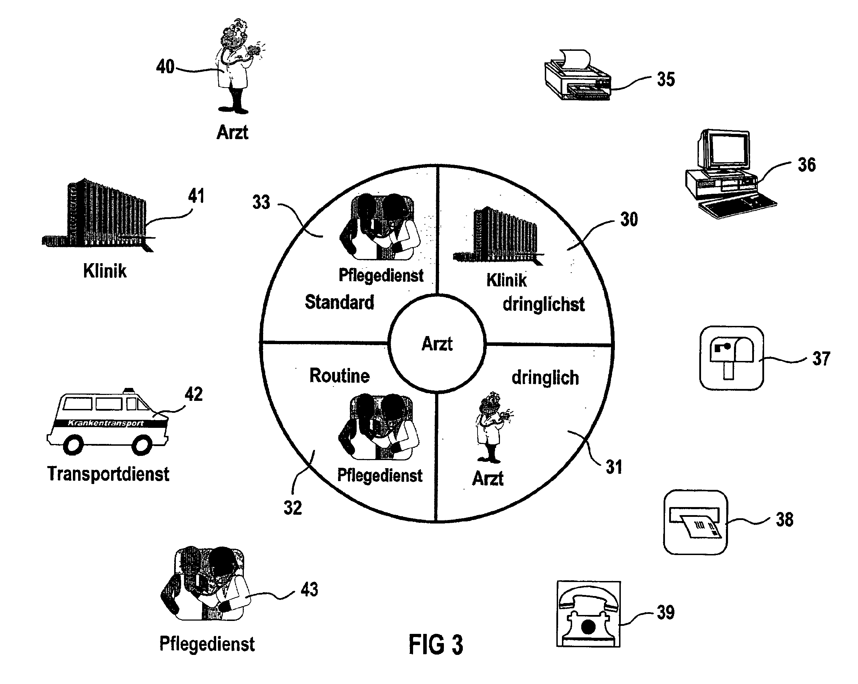

Wenn ein Arzt auf einen Alarm nicht in angemessener Zeit reagiert, leitet der Alarmgeber der Auswertevorrichtung 12 in der Systemzentrale 6 den Alarm weiter. Die Konfiguration, an wen der Alarm weitergeleitet werden sollen, trifft der Arzt selbst. Er verwendet hierzu einen in Fig. 3 dargestellten Kommunikationskreis, der anhand der Dringlichkeitsstufen segmentiert ist. Er zieht in die entsprechenden Kreissegmente die Ärzte und/oder Institutionen 40 bis 43 hinein, die im Notfall zu alarmieren sind, wenn er selbst nicht reagiert.

Bei der gewählten Konfiguration gemäß Fig. 3 wird bei dringlichsten Fällen eine Klinik 41, bei dringlichen Fällen ein anderer ausgewählter Arzt 40 und in allen anderen Fällen der Pflegedienst benachrichtigt.

Es ist zusätzlich vorgesehen, dass z.B. die Alarmierung des Arztes selbst und die ggf. erforderliche Weiterleitung in einem einzigen Kommunikationskreis erfolgen.

Es ist zusätzlich vorgesehen, dass Default-Einstellungen vom Server vorgegeben werden, so dass beim Neueinrichten dem Arzt ein sinnvoller Vorschlag vorgegeben wird, wobei eine Präferenzliste des Arztes berücksichtigt wird.

Weiterhin können unterschiedlich komplexe Alarme generiert werden. Im einfachsten Fall werden nur der Arzt oder seine Vertreter informiert. Die Alarme können aber auch krankheitsspezifisch an unterschiedliche Stellen weitergeleitet werden. So können die Medikamenteneinstellung betreffende Alarme an einen behandelnden Facharzt und akute Notfälle infolge von stark von der Norm abweichender Blutgerinnungswerte an eine klinische Einrichtung weitergeleitet werden. Die Alarme veranlassen zusätzlich erforderliche Maßnahmen. Beispielsweise alarmieren sie bei Verdacht innerer Blutungen den behandelnden Arzt und gleichzeitig die Feuerwehrleitstelle und den Transportdienst von der Notfallkette.

Auch der Informationskanal zum Patienten lässt sich konfigurieren, so dass auch der Patient vom Arzt Nachrichten empfangen kann. Dazu kann vom Patienten ebenfalls ein Kommunikationskreis konfiguriert werden, in den er seine Erreichbarkeitswege einträgt.

Ebenso ist vorgesehen, dass der Patient eine Weiterleitung dringender Nachrichten im Urlaub oder Krankenhausaufenthalt einstellen kann.

Das Versenden von Informationen geht dabei derart vonstatten, dass die Auswertevorrichtung 12 eine Nachricht erzeugt und diese an den Kommunikations-Server 14 zum Nachrichtenversand weitergibt. Der Kommunikations-Server 14 als Routingvorrichtung versendet Nachrichten an einen Empfänger unter Zuhilfenahme einer Unterkomponente Telefonbuch, in dem die Kontaktinformationen zu einem adressierbaren Empfänger bereitgestellt werden.

Die Endgeräte bei den Ärzten dienen zur Wiedergabe und Darstellung der Messwerte sowie der Parametrierung des Alarmgebers, der Benachrichtigungswege für Nachrichtenversand und der Endgeräte für Patient und/oder Pfleger.

Die Alarm-Endgeräte dienen auch der Ausgabe der vom Nachrichtenversand versandten Nachrichten beim Empfänger. Hierzu können spezielle Ausführung der Endgeräte für Patienten und/oder Pflegepersonen dienen wie PDA mit Modem, Telefon mit Sprache oder Tonwahl (DTMF), Messgerät mit integriertem Modem, TV Set-Top-Box, Handy, WWW-Formular oder Papierformular und WWW-Formular.

Die Systemzentrale 6 besitzt eine Schnittstelle zur Kopplung mit einem Qualitätssicherungssystem, um eine Ergebnisqualität unter Realbedingungen zu erhalten. Weiterhin kann das zentrale System eine Schnittstelle zur Kopplung mit einem Abrechnungsmodul aufweisen.

Spezielle Ausführungen des Alarmgebers können eine Alarmauslösung bei fehlerhaft erfassten Messwerten, eine Alarmauslösung bei Compliance-Problemen des Patienten, eine Alarmauslösung bei zu langer Reaktionszeit des Arztes auf dringende Nachrichten und eine Alarmweiterleitung vorsehen, falls der Arzt nicht reagiert.

Nachrichten können beispielsweise über Sprachausgabe per Telefon, SMS, E-Mail, WWW, WAP, Fax, Proprietärer Dienst oder Briefpost übermittelt werden.

Die Auswertevorrichtung 12 für die Messwerte und der Patientendaten-Server 13 können zur Datenerhebung so ausgeführt sein, dass die Messwerte sicher und in pseudonymer Form gespeichert werden.

Die Endgeräte für Ärzte weisen vorzugsweise eine auf Internet-Technologien basierende grafische Benutzeroberfläche auf. Zur Parametrierung des Alarmgebers bietet das Gerät das in Fig. 2 visualisierte Verfahren an. Zur Parametrierung der Benachrichtigungswege dient das anhand Fig. 3 dargestellte Verfahren.

Das Alarm-Endgerät kann je nach Alarmstufe beispielsweise ein Telefon, Handy, E-Mail-Terminal, WWW-Terminal, Fax-Gerät, Terminal zur Nutzung eines proprietären Diensts oder auch der Briefkasten sein.

Das Alarm-Endgerät in der Gemeinschaftspraxis 23 kann einen Rückkanal 29 zur Übertragung von Therapie-Hinweisen an den Patient und/oder Pfleger aufweisen, so dass eine Therapie trotz räumlicher Trennung von Arzt und Patient durchgeführt werden kann (Teletherapie).

Durch das erfindungsgemäße System wird eine "virtuelle" Krankenstation beim Patienten zu Hause geschaffen, die ein zentrales Alarmgebersystem aufweist, das Alarme generiert bei:
- Mangelhafter Patientencompliance (erwartete Messwerte des Patienten bleiben aus)
- Überschreiten von Schwellwerten (d.h. die Messwerte liegen in einem abnormen Bereich)
- Mangelhafter Arztcompliance (erwartete Reaktion auf Alarm durch den Arzt bleibt aus).

Ein Konfigurationskonzept für die Erreichbarkeit und die Alarmweiterleitung ermöglicht die Einstellungen nach den aktuellen Bedürfnissen jedes Betreuungsfalles.
- Eskalatationsmechanismen sorgen bei fehlender Reaktion eines Arztes für eine Weiterleitung.
- Es lassen sich krankheitsspezifische Alarmweiterleitungen einstellen.
- Es kann eine Behandlungskette wie Notarzt, Transport, Klinik, Pflegedienst und/oder Reha alarmiert und koordiniert werden.
- Eine Konfiguration der Datenkommunikation vom Arzt und/oder Pfleger zum Patienten kann durch den Patienten erreicht werden.

## Patentansprüche

1. Medizinisches System zur Überwachung eines die Blutgerinnung kennzeichnenden Messwertes eines Patienten und/oder zur Einstellung eines die Blutgerinnung beeinflussenden Medikamentes für einen Patienten in häuslicher Umgebung mit einer Vorrichtung (2) zur Erfassung von die Blutgerinnung betreffenden Messwerten des Patienten, einer Vorrichtung (2 bis 8) zur Übertragung der Messwerte an eine Systemzentrale (6), mit einer Vorrichtung (20 bis 22, 24) zur Abfrage der Messwerte und einer Empfangsvorrichtung (20 bis 22, 24) bei einer überwachenden Person, wobei die Systemzentrale (6) einen Speicher (15) für die Messwerte, eine Auswertevorrichtung (12) für die Messwerte zum Vergleich der Messwerte mit gespeicherten Sollwerten, einen Alarmgeber (12) zur Erzeugung eines Alarmsignals und eine Routingvorrichtung (14) zur Weiterleitung des Alarmsignals an eine Empfangsvorrichtung (3 bis 5, 20 bis 22, 24) einer zu alarmierenden Person einer Prozesskette aufweist.

2. Medizinisches System nach Anspruch 1, dessen Alarmgeber (12) derart ausgebildet ist, dass er ein Alarmsignal bei Über- oder Unterschreitung der Messwerte von gespeicherten Sollwertgrenzen erzeugt, das an eine Empfangsvorrichtung (20 bis 22, 24) einer Person einer Behandlungskette geleitet wird.

3. Medizinisches System nach Anspruch 1 oder 2, dessen Alarmgeber (12) ein Alarmsignal bei signifikanter Über- oder Unterschreitung der Messwerte von gespeicherten Sollwertgrenzen erzeugt, das an eine Empfangsvorrichtung (20 bis 22, 24) einer Person einer Notfallkette geleitet wird.

4. Medizinisches System nach einem der Ansprüche 1 bis 3, dessen Alarmgeber (12) derart ausgebildet ist, dass er ein Alarmsignal bei Ausbleiben von Messwerten erzeugt, das an eine Empfangsvorrichtung (3 bis 5) des Patienten geleitet wird.

5. Medizinisches System nach einem der Ansprüche 1 bis 4, dessen Alarmgeber (12) derart ausgebildet ist, dass er ein Alarmsignal bei Ausbleiben einer Reaktion einer Person einer Behandlungskette auf besonders gekennzeichnete Werte erzeugt, das an eine Empfangsvorrichtung (20 bis 22, 24) einer Notfallkette geleitet wird.

6. Medizinisches System nach einem der Ansprüche 1 bis 5, dessen Routingvorrichtung (14) derart ausgebildet ist, dass das Alarmsignal an eine vorbestimmte, auswählbare Empfangsvorrichtung (3 bis 5, 20 bis 22, 24) der Prozesskette geleitet wird.

7. Medizinisches System nach einem der Ansprüche 1 bis 6, dessen Routingvorrichtung (14) derart ausgebildet ist, dass das Alarmsignal durch automatisches Routing an eine von der Routingvorrichtung (14) unter Berücksichtigung der Verfügbarkeit bestimmte Empfangsvorrichtung (3 bis 5, 20 bis 22, 24) der Prozesskette geleitet wird.

8. Medizinisches System nach einem der Ansprüche 1 bis 7, dessen Routingvorrichtung (14) ein lernendes Expertensystem aufweist, das die Alarmierung der Prozesskette bewirkt.

9. Medizinisches System nach einem der Ansprüche 1 bis 8, dessen Auswertevorrichtung (12) ein lernendes Expertensystem aufweist, das die Messwerte krankheits- und/oder problemspezifisch anhand von Regelsystemen oder auf Wahrscheinlichkeiten basierend interpretiert, aufgrund deren Ergebnis das Alarmsignal ausgelöst wird.

10. Medizinisches System nach einem der Ansprüche 1 bis 9, dessen Alarmgeber (12) derart ausgebildet ist, dass er in Abhängigkeit von der Dringlichkeit einer Reaktion unterschiedliche Alarmsignale erzeugt, die an Empfangsvorrichtungen (3 bis 5, 20 bis 22, 24) einer Behandlungs- und/oder Notfallkette geleitet werden.
